Europäisches Patentamt

(19) European Patent Office

Office européen des brevets

(11) Veröffentlichungsnummer: **0 229 884**
B1

(12) **EUROPÄISCHE PATENTSCHRIFT**

(45) Veröffentlichungstag der Patentschrift:
03.05.89

(51) Int. Cl.⁴: **C07D 307/20**

(21) Anmeldenummer: **86114053.1**

(22) Anmeldetag: **10.10.86**

(54) Verfahren zur Herstellung von 2,5-Dimethoxy-und 2,5-Diethoxytetrahydrofuran.

(30) Priorität: **13.12.85 DE 3544046**

(43) Veröffentlichungstag der Anmeldung:
**29.07.87 Patentblatt 87/31**

(45) Bekanntmachung des Hinweises auf die Patenterteilung:
**03.05.89 Patentblatt 89/18**

(84) Benannte Vertragsstaaten:
**AT BE CH DE ES FR GB IT LI NL SE**

(56) Entgegenhaltungen:
**EP-A- 0 147 593**
**US-A- 2 920 081**
**US-A- 4 064 145**
**US-A- 4 380 657**

**CHEMICAL ABSTRACTS, Band 59, no. 9, 28.**
**Oktober 1963, Columbus, Ohio, USA, M. KRATOCHVIL**
**AND J. HORT "Methyl tetrahydrofuryl ethers",**
**Zusammenfassung-Nr. 9985b**
**HOUBEN-WEYL "Methoden der organischen**
**Chemie", 4. Auflage, Band VI/3,**
**Sauerstoffverbindungen I, 1965, GEORG THIEME**
**VERLAG, Stuttgart, pp. 539-541**

(73) Patentinhaber: **Degussa Aktiengesellschaft,**
**Weissfrauenstrasse 9, D-6000 Frankfurt am Main 1(DE)**

(72) Erfinder: **Andrade, Juan, Dr. Dipl.-Chem., 545 Barnett**
**Place, Ridgewood N.J. 07450(US)**
Erfinder: **Prescher, Günter, Dr. Dipl.-Chem.,**
**Liesingstrasse 2, D-6450 Hanau 9(DE)**

**Beschreibung**

Die Erfindung betrifft ein Verfahren zur Herstellung von 2,5-Dimethoxy- und 2,5-Diethoxytetrahydrofuran. Es ist bekannt, diese Verbindungen herzustellen, in dem man 1,5,9-Cyclododecatrien mit Ozon und Methanol oder Ethanol behandelt und das hierbei entstehende Ozonisierungsprodukt durch Hydrierung und anschließende Destillation zum 2,5-Dimethoxy- oder 2,5-Diethoxytetrahydrofuran umsetzt (JP-PS 19930).

Nachteilig ist bei diesem Verfahren, daß man ein Gemisch aus ca. 60% Succinaldehydtetramethyl- oder tetraethylacetal und 40% 2,5-Dimethoxy- oder 2,5-Diethoxytetrahydrofuran erhält.

Es ist auch bekannt, Tetrahydrofuran zu chlorieren und das hierbei entstehende 2,5-Dichlortetrahydrofuran mit Natriummethylat oder Natriumethylat zu den gewünschten Dialkoxyetrahydrofuran umzuwandeln (DDR-PS 25656).

Nachteilig bei diesem Verfahren ist die schlechte Ausbeute bei der Chlorierung.

Es ist weiterhin bekannt, von Furan ausgehend zu 2,5-Dialkoxytetrahydrofuranen zu gelangen. Hierzu wird aus Furan durch Reaktion mit Brom und dem entsprechenden Alkohol 2,5-Dialkoxydihydrofuran gebildet, und dieses zum 2,5-Dialkoxytetrahydrofuran hydriert (J. Am. Chem. Soc., 72, 869 (1950)).

In der US 2 920 081 wird ein Verfahren beschrieben, bei dem man 2,5-Dialkoxytetrahydrofurane durch die Umsetzung von 4,4-Diacyloxybutanalen und niedrigen Alkoholen in Gegenwart eines stark sauren Katalysators gewinnt.

Spurenmengen von 2,5-Dimethoxytetrahydrofuran erhält man, indem man 1,5-Cyclooctadien ozonisiert, das dabei erhaltene Produkt in Gegenwart eines Pt-Katalysators hydriert, die Reaktionslösung vom Katalysator befreit und den Rückstand unter saurer Katalyse acetalisiert (EP-A 0 147 593).

Es wurde nun ein einfaches Verfahren zur Herstellung der gewünschten Verbindungen gefunden, das dadurch gekennzeichnet ist, daß man 4,4-Dimethoxy- bzw. 4,4-Diethoxy-1-butanal bei einer Temperatur zwischen 0 und 70°C mit einem stark sauren Ionenaustauscherharz behandelt.

Die Behandlung mit dem Ionenaustauscherharz wird vorzugsweise bei einer Temperatur zwischen 20 und 60°C vorgenommen.

Man setzt bevorzugt je Gewichtsteil des Butanals 0,01 bis 0,1 Gewichtsteil des stark sauren Ionenaustauschers ein.

Die als Ausgangsmaterial dienenden 4,4-Dialkoxybutanale können beispielsweise gemäß dem in der DE-OS 3 403 427 beschriebenen Verfahren gewonnen werden. Hierbei wird Acrolein mit einem Alkanol zu einem 3,3-Dialkoxy-1-propen umgesetzt und dieses in Gegenwart von Hydridotris-triphenylphosphin-rodiumcarbonyl sowie Triphenylphosphit als Katalysator hydroformyliert.

Die geeigneten stark sauren Ionenaustauscherharze bestehen bevorzugt aus Styrol-Divenylbenzol-Mischpolymerisaten, die Sulfo-Gruppen als funktionelle Bestandteile tragen. Handelsüblich sind beispielsweise Dowex MSC-1, Amberlite IR-200C.

Das erfindungsgemäße Verfahren kann beispielsweise so durchgeführt werden, daß man das 4,4-Dialkoxy-1-butanal bei einer Temperatur zwischen 20 und 60°C in Gegenwart des Ionenaustauschers ausreichend lange kräftig rührt. Nach Abschluß der Reaktion erfolgt die Aufarbeitung des Gemisches beispielsweise so, daß man das Ionenaustauscherharz abfiltriert und das Reaktionsgemisch fraktionierend destilliert.

Die Erfindung wird durch die nachfolgenden Beispiele näher erläutert:

<u>Beispiele</u>

Beispiel 1

Es werden 50 g (0,38 Mol) 4,4-Dimethoxy-1-butanal mit 5 g stark saurem Ionenaustauscherharz (Dowex MSC-1) vermischt und 3 Stunden bei 60°C gerührt. Danach sind 74% des eingesetzten Butanals umgesetzt. Anschließend wird das Ionenaustauscherharz abfiltriert und das Reaktionsgemisch unter vermindertem Druck destilliert. Bei ca. 80°C Kopftemperatur (100 mbar) gingen 31,3 g 2,5-Dimethoxytetrahydrofuran über. Das Produkt besteht zu 97,6% aus den cis- bzw. trans-Isomeren der gewünschten Verbindung. Die Ausbeute, bezogen auf das umgesetzte 4,4-Dimethoxybutanal beträgt somit 82,6%.

Beispiel 2

Es werden 32 g (0,2 Mol) 4,4-Diethoxy-1-butanal mit 1,0 g stark saurem Ionenaustauscherharz (Amberlite IR-200c) vermischt und 2 Stunden bei 40°C gerührt. Danach waren 86% des eingesetzten Butanals umgesetzt. Anschließend wird das Ionenaustauscherharz abfiltriert und das Filtrat unter vermindertem Druck destilliert. Bei 64 bis 67°C Kopftemperatur (20 mbar) gehen 24,7 g 2,5-Diethoxytetrahydrofuran über. Das Produkt (ein Gemisch von cis- und trans-Isomer) ist 96,6%ig. Die Ausbeute, bezogen auf das umgesetzte Butanal beträgt 86,7%.

Beispiel 3

Es wird wie im Beispiel 1 verfahren, jedoch wird eine Mischung von 132 g (1,0 Mol) 4,4-Dimethoxy-1-butanal und 2,6 g Ionenaustauscherharz (Amberlite IR-200c) eingesetzt und die Mischung 1,5 Stunden bei 50°C gerührt. Der Umsatz beläuft sich auf 94%. Das Produkt besteht zu 98% aus den cis- bzw. trans-Isomeren des 2,5-Dimethoxytetrahydrofurans. Die Verbindung siedet bei 144 bis 147°C. Die Ausbeute, bezogen auf das umgesetzte Butanal beträgt 95,2%.

Beispiel 4

Es wird in Beispiel 2 verfahren, jedoch eine Mischung von 80 g (0,5 Mol) 4,4-Diethoxy-1-butanal und 4 g Ionenaustauscherharz (Dowex MSC-1) eingesetzt und 4 Stunden bei 30°C gerührt. Der Umsatz beträgt 82%. Nach der Abtrennung des Ionenaustauschers geht bei 63 bis 65°C (20 mbar) ein Produkt über, das zu 97,5% aus den cis- bzw. trans-Isomeren des 2,5-Diethoxytetrahydrofurans besteht. Die Ausbeute, bezogen auf das eingesetzte Butanal beläuft sich auf 91%.

**Patentansprüche**

1. Verfahren zur Herstellung von 2,5-Dimethoxy- und 2,5-Diethoxytetrahydrofuran, dadurch gekennzeichnet, daß man ein 4,4-Dimethoxy- oder 4,4-Diethoxy-1-butanal bei einer Temperatur zwischen 0 und 70°C mit einem stark sauren Ionenaustauscherharz behandelt.

2. Verfahren nach Anspruch 1, dadurch gekennzeichnet, daß man je Gewichtsteil des Butanals 0,001 bis 0,1 Gewichtsteile des stark sauren Ionenaustauschers verwendet.

3. Verfahren nach Anspruch 1 oder 2, dadurch gekennzeichnet, daß man die Reaktion bei einer Temperatur zwischen 20 und 60°C vornimmt.

**Claims**

1. Process for the preparation of 2,5-dimethoxy- and 2,5-diethoxytetrahydrofuran, characterized in that a 4,4-dimethoxy- or 4,4-diethoxy-1-butanal is treated at a temperature between 0 and 70°C with a strongly acidic ion-exchanger resin.

2. Process according to Claim 1, characterized in that 0.001 to 0.1 part by weight of the strongly acidic ion-exchanger is used per part by weight of butanal.

3. Process according to Claim 1 or 2, characterized in that the reaction is carried out at a temperature between 20 and 60°C.

**Revendications**

1. Procédé pour la préparation du 2,5-diméthoxy- et du 2,5-diéthoxytétrahydrofuranne, caractérisé en ce que l'on traite un 4,4-diméthoxy- ou 4,4-diéthoxy-1-butanal, à une température comprise entre 0 et 70°C, avec une résine échangeuse d'ions fortement acide.

2. Procédé selon la revendication 1, caractérisé en ce que l'on utilise de 0,001 à 0,1 partie en poids de l'échangeur ionique fortement acide, par partie en poids du butanal.

3. Procédé selon la revendication 1 ou 2, caractérisé en ce que l'on effectue la réaction à une température comprise entre 20 et 60°C.